# EUROPÄISCHE PATENTANMELDUNG

(11) **EP 2 975 019 A1**
(43) Veröffentlichungstag der Anmeldung: **20.01.2016**
(21) Anmeldenummer: 15174717.7
(22) Anmeldetag: 01.07.2015
(51) Int. Cl.: C07C 209/60, C07C 213/02, C07C 227/10, C07C 253/30, C07F 15/02, C07F 9/50, B01J 31/24

(54) **RUTHENIUM-KATALYSIERTE HYDROAMINOMETHYLIERUNG**

(30) Priorität: 18.07.2014 DE 102014214030
(71) Anmelder: Evonik Degussa GmbH, 45128 Essen (DE)
(72) Erfinder: Franke, Robert, 45772 Marl (DE); Dyballa, Katrin Marie, 45657 Recklinghausen (DE); Beller, Matthias, 18211 Nienhagen (DE); Jackstell, Ralf, 27478 Cuxhaven Altenwalde (DE); Liu, Qiang, 300161 Hedong District Tianjin (CN); Zhang, Min, 510600 Yuexiu district, Guangzhou (CN); Wu, Lipeng, 055150 City Xingtai (CN); Fleischer, Ivana, 93057 Regensburg (DE)

(57) **Zusammenfassung**

Verfahren zur Hydroaminomethylierung von Verbindungen, welche mindestens eine Doppelbindung enthalten, wobei eine Verbindung, welche mindestens eine Doppelbindung enthält, mit Ammoniak oder einer Ammoniakquelle unter Zuführung von H₂ und CO in Gegenwart eines katalytischen Systems aus einem Ruthenium-Komplex und eines organischen PhosphorLiganden umgesetzt wird.

## Beschreibung

Die vorliegende Erfindung betrifft ein Verfahren zur Hydroaminomethylierung von Verbindungen, welche mindestens eine Doppelbindung enthalten, aus einem solchen Verfahren resultierende Gemische sowie die Verwendung des in den obigen Verfahren eingesetzen Liganden bei Umsetzungen von Ammoniak oder einer Ammoniakquelle mit Verbindungen, welche mindestens eine Doppelbindung enthalten.

Amine werden in einigen Millionen Tonnen pro Jahr produziert und finden z.B. als pharmakologisch und biologisch aktive Substanzen, als Farbstoffe, Agrochemikalien oder als Feinchemikalien industrielle Verwendung. Die zur Verfügung stehenden Synthesemethoden sind jedoch oft mit erheblichen Nachteilen verbunden. So sind z.B. die Ausgangssubstanzen für die Aminsynthese sehr teuer oder bei der Reaktion fällt eine große Menge an Nebenprodukten an oder zur Durchführung der Reaktion müssen Schutzgruppen eingeführt und wieder entfernt werden. Aus ökonomischen wie auch aus ökologischen Gründen besteht daher ein dringendes Bedürfnis, einfach umzusetzende Verfahren zur selektiven Herstellung aliphatischer Amine ausgehend von kostengünstigen Ausgangsmaterialien bereitzustellen.

Insbesondere die Synthese von Aminen aus an sich gut verfügbaren Olefinen ist von besonderem Interesse. Hier stellt die so genannte Hydroaminomethylierung eine umweltfreundliche Herstellung von Aminen dar. Seit der Entdeckung dieser Reaktion durch W. Reppe (Reppe, W.; Vetter, H. Liebigs Ann. Chem. 1953, 582, 133-163), wurde die Hydroaminomethylierungsreaktion weiter erforscht. Eine von L. F. Tietze (Chem. Rev. 1996, 96, 115-136) und G. H. Posner (Chem. Rev. 1986, 86, 831-844) beschriebene "Domino-Reaktion" umfasst die Hydroformylierung des Olefins zum Aldehyd, danach Aminierung des Aldehyds zum Enamin oder Imin sowie die anschließende Hydrierung des Zwischenprodukts:

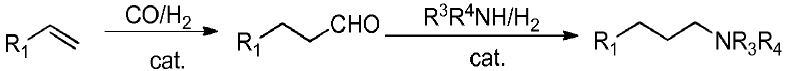

Die Herstellung von tertiären Aminen durch Hydroaminomethylierung von Olefinen mit mindestens einem primären oder sekundären Aminen unter Zuführung von H₂ und CO unter katalytischen Bedingungen in Gegenwart eines Ruthenium-Komplexes und eines organischen Phosphorliganden wird in "Efficient and regioselective Ruthenium-catalyzed hydroaminomethylation of olefins" von Lipeng Wu, Ivana Fleischer, Ralf Jackstell, Matthias Beller, J. Am. Chem. Soc. 2013, 135, 10, 3989-3996 beschrieben.

Die phosphorhaltigen Liganden lassen sich gemäß dem Fachmann bekannten Syntheseverfahren, wie sie z. B. nach Methoden wie sie in Chemistry of Organophosphorous Compounds, Ed. F. R. Hartley, Serial Ed. S. Patai, Vol. 1, John Whiley, 1990 oder in "Phosphorous(III)Ligands in Homogeneous Catalysis - Design and Synthesis" von Paul C.J. Kamer und Piet W.N.M. van Leeuwen; John Wiley and Sons, 2012 sowie darin enthaltene Literaturhinweise beschrieben sind, herstellen.

Die Herstellung von tertiären Aminen durch Hydroaminomethylierung von Olefinen in Gegenwart eines Ruthenium-Komplexes ohne Ligandenzusatz wird in "Phosphine- and hydrogenfree highly regioselective Ruthenium-catalyzed hydroaminomethylation of olefins" von Samet Gülak, Lipeng Wu, Qiang Liu, Robert Franke, Ralf Jackstell, Matthias Beller, Angew. Chem. Int. Ed. 2014, 513, 7320-7323 beschrieben.

Von weitaus größerem Interesse ist jedoch die Synthese von Aminen direkt ausgehend von Ammoniak oder einer Ammoniakquelle, da diese gut verfügbar und kostengünstiger als die entsprechenden Amine sind.

Die meisten industriellen Prozesse, bei denen Ammoniak zum Einsatz kommt, erfordern allerdings drastische Reaktionsbedingungen wie hohe Temperatur, Druck und benötigen eine sehr spezielle und kostenintensive Ausstattung für den Einsatz von gasförmigem Ammoniak unter diesen Bedingungen.

Um Ammoniak unter milderen Bedingungen umsetzen zu können, wurden verschiedene katalytische Umsetzungen entwickelt, welche organometallische Komplexe nutzen. Der Einsatz von Ammoniak als Stickstoffquelle erweist sich in den Punkten Katalysatoraktivität, Chemo- und Regioselektivität jedoch als schwierig. Insbesondere aufgrund der wachsenden sterischen Hinderung (in der Reihe Ammoniak < primäres Amin < sekundäres Amin) liefern typische Hydroformylierungskatalysatoren bei der Umsetzung mit Ammoniak - in Abhängigkeit vom jeweiligen Substrat - stets ein Gemisch von primären, sekundären und tertiären Aminen.

So erhielt J. F. Knifton bei einer Phosphin-Ligand-Cobalt-katalysierten Hydroaminomethylierung von 1-Hexen mit Ammoniak Mischungen von primären und sekundären Aminen (J.F. Knifton, Catal. Today 1997, 36, 305-310) und die bimetallische Rhodium/Iridium-katalysierte Hydroaminometallierung von niedrigen aliphatischen Olefinen mit Ammoniak unter Einsatz eines zweiphasigen Systems und eines wasserlöslichen trisulfoniertem Triphenylphosphion-Liganden lieferte mit 90 %-iger Chemoselektivität hauptsächlich primäre Amine siehe hierzu: (a) B. Zimmermann, J. Herwig, M. Beller, Angew. Chem. Int. Ed. 1999, 38, 2372-2375; b) H. Klein, R. Jackstell, M. Kant, A. Martin, M. Beller, Chem. Eng. Technol. 2007, 30, 721-725).

Jenseits von Rhodium- und Cobalt-Komplexen wurde die Eignung anderer Metallkatalysatorsysteme in Hydroaminomethylierungsreaktionen, insbesondere in Gegenwart von Ammoniak oder einer Ammoniakquelle, wenig untersucht.

Aufgabe der vorliegenden Erfindung war es daher, ein Verfahren zur Hydroaminomethylierung von leicht zugänglichen Olefinen (Alkenen) zur Verfügung zu stellen, das insbesondere die oben genannten Nachteile vermeiden kann. Dieses Verfahren sollte direkt von Ammoniak oder einer Ammoniakquelle ausgehen.

Die Aufgabe wurde gelöst durch ein Hydroaminomethylierungs-Verfahren, in welchem Verbindungen, welche mindestens eine Doppelbindung enthalten, mit Ammoniak oder einer Ammoniakquelle unter Zuführung von H₂ und CO umgesetzt werden, wobei ein Ligand-Ruthenium-Komplex als Katalysator eingesetzt wird. Überraschend ist dieses Verfahren effizient und weist zudem eine hohe Regioselektivität auf.

Gegenstand der vorliegenden Erfindung ist ein Verfahren zur Hydroaminomethylierung von Verbindungen, welche mindestens eine Doppelbindung enthalten, dadurch gekennzeichnet, dass wenigstens eine Verbindung, welche mindestens eine Doppelbindung enthält, mit Ammoniak oder einer Ammoniakquelle unter Zuführung von H₂ und CO in Gegenwart eines katalytischen Systems aus einem Ruthenium-Komplex und mindestens einem Liganden umgesetzt wird, wobei der Ligand einen organischen Phosphor-Liganden einer der allgemeinen Formeln (1) bis (8) darstellt: 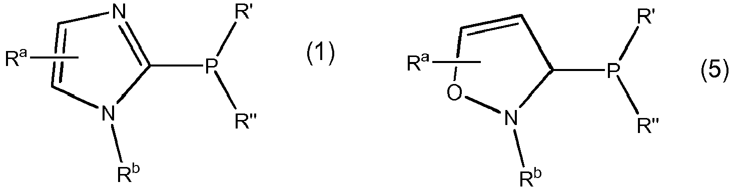 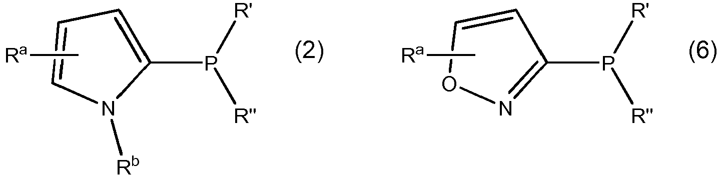 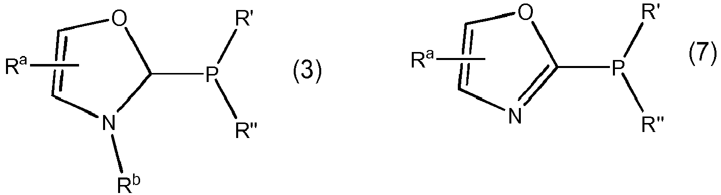 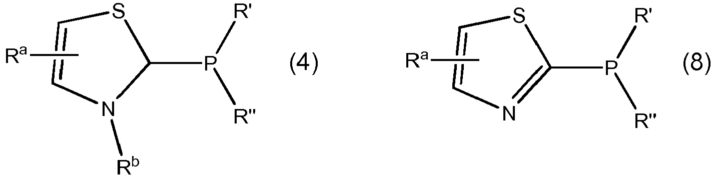 worin
R' und R" gleich oder verschieden sind und für einen Rest stehen, der ausgewählt ist aus der Gruppe bestehend aus:
(C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
   wobei die genannten Alkyl-, Heteroalkyl-, Cycloalkyl-, Heterocycloalkyl-, Aryl- und Heteroarylgruppen optional ein oder mehrfach substituiert sind und
R^{a} und R^{b} ausgewählt sind aus: Wasserstoff, Hydroxy, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
   wobei die genannten Alkyl-, Heteroalkyl-, Cycloalkyl-, Heterocycloalkyl-, Aryl- und Heteroarylgruppen optional ein oder mehrfach substituiert sind und
   und wobei R^{a} auch zur Bildung eines größeren kondensierten Ringes befähigt ist.

Alkyl steht für einen nicht verzweigten oder verzweigten aliphatischen Rest, Aryl für aromatische (Kohlenwasserstoff-)Reste, vorzugsweise mit bis zu 14 C-Atomen, z. B. Phenyl- (C₆H₅-), Naphthyl- (C₁₀H₇-), Anthryl- (C₁₄H₉-), vorzugsweise Phenyl. Cycloalkyl steht für gesättigte cyclische Kohlenwasserstoffe, die ausschließlich Kohlenstoff-Atome im Ring enthalten, Heteroalkyl für einen nicht verzweigten oder verzweigten aliphatischen Rest, der ein bis vier, bevorzugt ein oder zwei Heteroatome ausgewählt aus der Gruppe bestehend aus N, O, S und substituiertem N enthalten kann. Heteroaryl steht für einen Arylrest, in dem ein bis vier, bevorzugt ein oder zwei, Kohlenstoffatome durch Heteroatome ausgewählt aus der Gruppe bestehend aus N, O, S und substituiertem N ersetzt sein können, wobei der Heteroarylrest auch Teil einer größeren kondensierten Ringstruktur sein kann. Heterocycloalkyl steht für gesättigte cyclische Kohlenwasserstoffe, die ein bis vier, bevorzugt ein oder zwei, Heteroatome ausgewählt aus der Gruppe bestehend aus N, O, S und substituiertem N enthalten kann. Unter Heteroarylrest, der Teil einer kondensierten Ringstruktur sein kann, werden bevorzugt Systeme verstanden, in denen kondensierte Fünf- oder Sechsringe gebildet werden, z.B. Benzofuran, Isobenzofuran, Indol, Isoindol, Benzothiophen, Benzo(c)thiophen, Benzimidazol, Purin, Indazol, Benzoxazol, Chinolin, Isochinolin, Chinoxalin, Chinazolin, Cinnolin, Acridin.

Die genannten substituierten N können einfach substituiert sein, die Alkyl-, Heteroalkyl-, Cycloalkyl-, Heterocycloalkyl-, Aryl- und Heteroarylgruppen können ein oder mehrfach, besonders bevorzugt ein-, zwei- oder dreifach substituiert sein durch Reste ausgewählt aus der Gruppe bestehend aus Wasserstoff, (C₁-C₁₄)-Alkyl, (C₁-C₁₄)-Heteroalkyl, (C₄-C₁₄)-Aryl, (C₄-C₁₄)-Aryl-(C₁-C₁₄)-Alkyl, (C₃-C₁₄)-Heteroaryl, (C₃-C₁₄)-Heteroaryl-(C₁-C₁₄)-Alkyl, (C₃-C₁₂)-Cycloalkyl, (C₃-C₁₂)-Cycloalkyl-(C₁-C₁₄)-Alkyl, (C₃-C₁₂)-Heterocycloalkyl, (C₃-C₁₂)-Heterocycloalkyl-(C₁-C₁₄)-Alkyl, CF₃, Halogen (Fluor, Chlor, Brom, lod), (C₁-C₁₀)-Haloalkyl, Hydroxy, (C₁-C₁₄)-Alkoxy, (C₄-C₁₄)-Aryloxy, O-(C₁-C₁₄)-Alkyl-(C₄-C₁₄)-Aryl, (C₃-C₁₄)-Heteroaryloxy, N((C₁-C₁₄)-Alkyl)₂, N((C₄-C₁₄)-Aryl)₂, N((C₁-C₁₄)-Alkyl)((C₄-C₁₄)-Aryl), wobei Alkyl, Aryl, Cycloalkyl, Heteroalkyl, Heteroaryl und Heterocycloalkyl die vorgenannten Bedeutungen haben.

In einer Variante des Verfahrens sind R' und R" ausgewählt aus: ggf. substituierten (C₄-C₁₄)-Aryl-Rest, einen ggf. substituierten unverzweigten oder verzweigten aliphatischen Kohlenwasserstoffrest oder einem ggf. substituierten Cycloalkylrest, und R^{a} und R^{b} sind ausgewählt aus: Wasserstoff, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, wobei die genannten Alkyl-, Heteroalkyl-, Cycloalkyl-, Heterocycloalkyl-, Aryl- und Heteroarylgruppen optional ein oder mehrfach substituiert sind und wobei R^{a} auch zur Bildung eines größeren kondensierten Ringes befähigt ist.

In einer anderen Variante des Verfahrens sind R' und R" ausgewählt aus: (C₁-C₈)-Alkyl, vorzugsweise Methyl, Ethyl, Propyl, *iso*-Propyl und *tert*-Butyl, (C₄-C₁₄)-Aryl-O-(C₁-C₈)-Alkyl, Aryl, vorzugsweise Phenyl, Cyclohexyl und R^{a} und R^{b} sind ausgewählt aus: Wasserstoff, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, wobei die genannten Alkyl-, Heteroalkyl-, Cycloalkyl-, Heterocycloalkyl-, Aryl- und Heteroarylgruppen optional ein oder mehrfach substituiert sind und wobei R^{a} auch zur Bildung eines größeren kondensierten Ringes befähigt ist.

In einer anderen Variante des Verfahrens sind R' und R" ausgewählt aus: C₁ bis C₈-Alkyl (insbesondere Methyl, Ethyl, Propyl, *iso*-Propyl und *tert*-Butyl), Phenyl, Cyclohexyl.

Eine Alkylgruppe hat bevorzugt 1-12, insbesondere 1 bis 6 Kohlenstoffatome. Alkylgruppen sind z.B. Methyl, Ethyl, Propyl, *iso*-Propyl, 1-Butyl, *tert*-Butyl, 1-Pentyl, 1-Hexyl.

In einer Ausführungsform ist R^{b} ausgewählt aus: Alkyl, Phenyl, (C₄-C₁₄)-Aryl-O-(C₁-C₁₂)-Alkyl und Alkyl-substituiertes Phenyl.

In einer Ausführungsform ist R^{a} gleich Wasserstoff.

Bevorzugt weist der Ligand die allgemeine Formel (1) oder (2) auf.

In einer Ausführungsform ist R' ausgewählt aus: Cyclohexyl, iso-Propyl, Phenyl.
In einer Ausführungsform ist R" ausgewählt aus: Cyclohexyl, iso-Propyl, Phenyl.
Bevorzugt ist R' und/oder R" - unabhängig voneinander - ausgewählt aus Cyclohexyl, *iso*-Propyl und Phenyl.

In einer Ausführungsform stehen R' und R" für den gleichen Rest

Besonders bevorzugt ist der Ligand ausgewählt ist aus der Gruppe bestehend aus:
2-(Dicyclohexylphosphino)-1-(2-methoxyphenyl)-1H-imidazol (L1),
2-(Dicyclohexylphosphino)-1-methyl-1 H-imidazol (L2),
2-(Dicyclohexylphosphino)-1-phenyl-1H-imidazol (L3),
2-(Di*iso*propylphosphino)-1-(2-methoxyphenyl)-1H-imidazol (L4),
2-(Diphenylphosphino)-1-(2-methoxyphenyl)-1 H-imidazol (L5),
2-(Dicyclohexylphosphino)-1-mesityl-1 H-imidazol (L6),
2-(Dicyclohexylphosphino)-1-phenyl-1 H-pyrrol (L7),
2-(Dicyclohexylphosphino)-1-(2-methoxyphenyl)-1H-pyrrol (L8) und
2-(Dicyclohexylphosphino)-1-methyl-1 H-benzo[d]imidazol (L9).

Insbesondere wird in dem Verfahren der Ligand 2-(Dicyclohexylphosphino)-1-(2-methoxyphenyl)-1 H-imidazol (L1) eingesetzt.

Erfindungsgemäß wird im vorliegenden Verfahren Ruthenium als Metallkatalysator eingesetzt.

In einer Variante des Verfahrens wird der Ruthenium-Katalysator in situ ausgehend von einem Vorkomplex gebildet, wobei als Rutheniumquelle Ruthenium-enthaltende Salze und Komplexe als Vorstufe verwendet werden, die Rutheniumcarbonylhydrid-Komplexe bilden, vorzugsweise Ru(0)-carbonylverbindungen, Ru(II)- und Ru(III)-halogenide.

Die Rutheniumverbindungen können in unterschiedlichen Oxidationsstufen vorliegen, die mit Synthesegas und P-enthaltenden Liganden zu entsprechenden aktiven Ruthenium-(hydrido)(carbonyl)-Komplexen reagieren.

Eine besonders bevorzugte Vorstufe zur Verwendung im erfindungsgemäßen Hydroaminomethylierungsverfahren sind Trirutheniumdodecacarbonyl [Ru₃(CO)₁₂] und Ru(methylallyl)₂(COD), insbesondere Trirutheniumdodecacarbonyl [Ru₃(CO)₁₂].

In einer Variante des Verfahrens wird der Ruthenium-haltige Vorkomplex in Mengen von 0,001 bis 10 mol-% in Bezug auf die Verbindung, welche mindestens eine Doppelbindung enthält, eingesetzt. Besonders bevorzugte Mengen liegen bei 0,05 bis 5 mol-% und insbesondere bei 0,1 bis 1 mol-% Vorkomplex in Bezug auf die Verbindung, welche mindestens eine Doppelbindung enthält.

In einer Variante des Verfahrens wird der Ligand in einem Verhältnis Ligand:Ruthenium zwischen 1:1 bis 4:1, vorzugsweise zwischen 1:1 und 2:1 eingesetzt.

Die Zielreaktion läuft vorzugsweise bei Temperaturen von 60 bis 180 °C ab; besonders bevorzugt bei 80 bis 160 °C, und bei einigen Ausführungsformen des Verfahrens bei 100 bis 140°C.

Im erfindungsgemäßen Verfahren werden Lösungsmittel für den Katalysator eingesetzt. Als Lösungsmittel werden im Allgemeinen polare inerte organische Lösungsmittel oder/und Wasser verwendet. Beispielhaft genannt werden dipolar aprotische Lösungsmittel, aliphatische Ether, Amide, aromatische Verbindungen, Alkohole und Ester, Ether sowie deren Gemische. In bevorzugten Ausführungsformen handelt es sich bei dem Lösungsmittel um beispielsweise N-Methyl-2-pyrrolidon (NMP), Methanol, Ethanol, *i*so-Propanol, Toluol, Tetrahydrofuran (THF), Acetonitril und Propylencarbonat (PC) oder Gemische derselben, beispielsweise um eine Mischung eines Alkohols mit einem Aromaten. Vorzugsweise werden in dem Verfahren als Lösungsmittel, Alkohole, beispielsweise Methanol, Ethanol, *i*so-Propanol oder Butanol, Acetonitril, Wasser, Toluol, THF, sowie Mischungen dieser Lösungsmittel, insbesondere ein THF/Toluol-Gemisch eingesetzt.

In einer anderen Ausführungsform wird weniger als 10 Massen-%, bevorzugt weniger als 5 Massen-%, besonders bevorzugt weniger als 2 Massen-%, jeweils bezogen auf das gesamte Reaktionsgemisch, und insbesondere kein Lösungsmittel in dem Verfahren eingesetzt.

In der Hydroaminomethylierung wird Ammoniak oder eine Ammoniakquelle eingesetzt. Unter dem Begriff Ammoniakquelle werden im Rahmen der vorliegenden Erfindung alle Verbindungen verstanden, welche unter geeigneten Bedingungen Ammoniak freisetzen können. Ein Beispiel für eine Ammoniakquelle ist Harnstoff. Vorzugsweise wird in dem Verfahren jedoch Ammoniak eingesetzt.

Der Ammoniak oder die Ammoniakquelle kann in dem Verfahren "in Substanz" oder als Gemisch mit einem Lösungsmittel eingesetzt werden. Geeignet sind alle Lösungsmittel, die bereits oben als Lösungsmittel für den Katalysator beschrieben sind. Bevorzugt wird der Ammoniak als wässrige Lösung oder "in Substanz" eingesetzt.

Bevorzugter Gegenstand der vorliegenden Erfindung ist ein Verfahren zur Hydroaminomethylierung von Verbindungen, welche mindestens eine Doppelbindung enthalten, dadurch gekennzeichnet, dass wenigstens eine Verbindung, welche mindestens eine Doppelbindung enthält, mit Ammoniak unter Zuführung von H₂ und CO in Gegenwart eines katalytischen Systems aus einem Ruthenium-Komplex und einem phosphorhaltigen Liganden umgesetzt wird, wobei
- Ammoniak als wässriger Ammoniak eingesetzt wird und/oder
- die Temperatur bei 80 bis 160 °C, insbesondere bei 100 bis 140 °C liegt und/oder,
- der Synthesegasdruck bei 10 bis 150 bar, insbesondere bei 20 bis 100 bar und insbesondere bei 30 bis 80 bar liegt,
- im Synthesegas das Verhältnis von CO : H₂ kleiner ist als 1:1, insbesondere kleiner ist als 1:2, und dabei vorzugsweise größer ist als 1:10, insbesondere größer ist als 1:6 und/oder
- weniger als 10 Massen.-% Lösungsmittel, bezogen auf das gesamte Reaktionsgemisch, enthalten sind und insbesondere kein Lösungsmittel enthalten ist,
- der Ruthenium-Komplex in situ, vorzugsweise aus [Ru₃(CO)₁₂] gebildet wird wobei mit Vorzug 0,05 bis 1,0 mol%, bevorzugt 0,1 bis 0,5 mol% [Ru₃(CO)₁₂] in Bezug auf das eingesetzte Olefin eingesetzt werden und/oder
- der Ligand ausgewählt ist aus
   - 2-(Dicyclohexylphosphino)-1-(2-methoxyphenyl)-1H-imidazol (L1),
   - 2-(Dicyclohexylphosphino)-1-methyl-1 H-imidazol (L2),
   - 2-(Dicyclohexylphosphino)-1-phenyl-1 H-imidazol (L3),
   - 2-(Di*i*sopropylphosphino)-1-(2-methoxyphenyl)-1H-imidazol (L4),
   - 2-(Diphenylphosphino)-1-(2-methoxyphenyl)-1 H-imidazol (L5),
   - 2-(Dicyclohexylphosphino)-1-mesityl-1 H-imidazol (L6),
   - 2-(Dicyclohexylphosphino)-1-phenyl-1 H-pyrrol (L7),
   - 2-(Dicyclohexylphosphino)-1-(2-methoxyphenyl)-1 H-pyrrol (L8),
   - 2-(Dicyclohexylphosphino)-1-methyl-1 H-benzo[d]imidazol (L9)
und insbesondere 2-(Dicyclohexylphosphino)-1-(2-methoxyphenyl)-1 H-imidazol ist.

In einer Variante des Verfahrens werden solche Verbindungen, welche mindestens eine Doppelbindung enthalten, zur Umsetzung verwendet, die eine oder mehrere ethylenisch ungesättigte Doppelbindungen enthalten, wie α-Alkene, interne lineare und interne verzweigte Alkene, Cycloalkene, aromatische Olefine und Polyene. In dem hier beschriebenen Hydroaminomethylierungs-Verfahren können auch funktionalisierte Alkene umgesetzt werden. Ohne Anspruch auf Vollständigkeit seien hier als Substrate ungesättigte Alkohole, Ether, Amine, Ester, Carbonsäuren, Amide, Urethane, Halogenide, Aldehyde, Ketone und Epoxide genannt. Besonders bevorzugt sind Carbonsäureester, welche mindestens eine Doppelbindung enthalten.

Eine Variante des erfindungsgemäßen Verfahrens zeichnet sich vorzugsweise dadurch aus, dass die Ausbeute an tertiären Aminen oberhalb von 50 Massen-%, vorzugsweise oberhalb von 60 Massen-%, weiter bevorzugt oberhalb von 70 Massen-% und insbesondere oberhalb von 80 Massen-%, bezogen auf das Produkt der Reaktion liegt.
Mit Vorzug wird eine Selektivität (*n*/*iso*) von größer 70:30, insbesondere von größer 80:20 erreicht.

Gegenstand der vorliegenden Erfindung sind zudem Gemische, welche durch ein der oben beschriebenen Verfahren erhalten wurden.

Weiterer Gegenstand der vorliegenden Erfindung ist die Verwendung eines organischen Phosphor-Liganden einer der allgemeinen Formeln (1) bis (8) 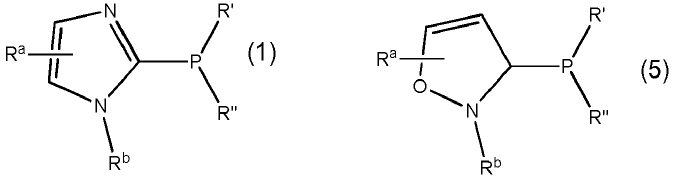 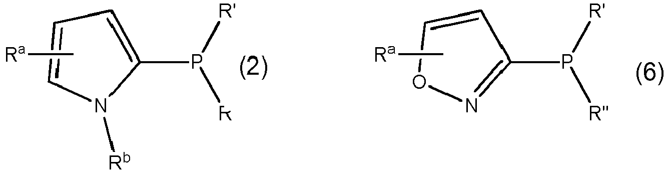 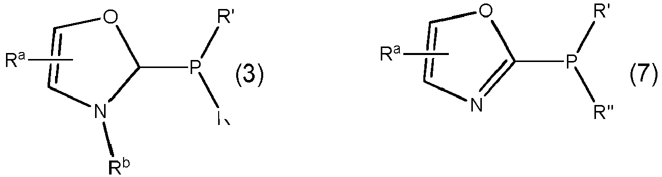 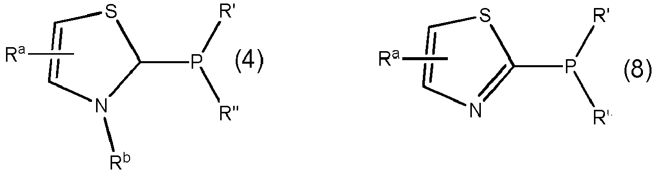 worin
R' und R" gleich oder verschieden sind und für einen Rest stehen, der ausgewählt ist aus der Gruppe bestehend aus:
(C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
   wobei die genannten Alkyl-, Heteroalkyl-, Cycloalkyl-, Heterocycloalkyl-, Aryl- und Heteroarylgruppen optional ein oder mehrfach substituiert sind und
R^{a} und R^{b} ausgewählt sind aus: Wasserstoff, Hydroxy, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl,O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
   wobei die genannten Alkyl-, Heteroalkyl-, Cycloalkyl-, Heterocycloalkyl-, Aryl- und Heteroarylgruppen optional ein oder mehrfach substituiert sind und
   und wobei R^{a} auch zur Bildung eines größeren kondensierten Ringes befähigt ist,
bei der Umsetzung von Ammoniak oder einer Ammoniakquelle mit Verbindungen, welche mindestens eine Doppelbindung enthalten.

Gegenstand der vorliegenden Erfindung ist zudem die Verwendung eines organischen Phosphorliganden aus der Gruppe, die gebildet wird durch
2-(Dicyclohexylphosphino)-1-(2-methoxyphenyl)-1H-imidazol,
2-(Dicyclohexylphosphino)-1-methyl-1H-imidazol,
2-(Dicyclohexylphosphino)-1-phenyl-1H-imidazol,
2-(Di*iso*propylphosphino)-1-(2-methoxyphenyl)-1H-imidazol,
2-(Diphenylphosphino)-1-(2-methoxyphenyl)-1H-imidazol,
2-(Dicyclohexylphosphino)-1-mesityl-1H-imidazol,
2-(Dicyclohexylphosphino)-1-phenyl-1H-pyrrol,
2-(Dicyclohexylphosphino)-1-(2-methoxyphenyl)-1H-pyrrol und
2-(Dicyclohexylphosphino)-1-methyl-1H-benzo[d]imidazol
bei der Umsetzung von Ammoniak oder einer Ammoniakquelle mit Verbindungen, welche mindestens eine Doppelbindung enthalten.

### Beispiele

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet oder zumindest entgast, um den Sauerstoff zu entfernen (Purification of Laboratory Chemicals, W. L. F. Armarego (Autor), Christina Chai (Autor), Butterworth Heinemann (Elsevier), 6. Auflage, Oxford 2009).
Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem. 2001, 73, 1795-1818; Robin K. Harris, Edwin D. Becker, Sonia M. Cabral de Menezes, Pierre Granger, Roy E. Hoffman and Kurt W. Zilm, Pure Appl. Chem. 2008, 80, 59-84).
Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten

Die bevorzugt verwendeten 2-Phosphino-substituierten heterocyclischen Liganden können z.B. gemäß Zhang, H.; Cai, Q.; Ma, D. J. Org. Chem. 2005, 70 (13), 5164-5173 oder Schulz, T.; Torborg, C.; Schäffner, B.; Huang, J.; Zapf, A.; Kadyrov, R.; Börner, A.; Beller, M. Angew. Chem. Int. Ed. 2009, 48, 918-921 hergestellt werden.

Ein entsprechend substituiertes -1*H*-Imidazol (13,5 mmol) wird unter Rückfluss in THF unter Argon gelöst und auf -30°C gekühlt. *n*-BuLi (1,6 M in Hexan, 8,4 ml, 13 mmol) wird zugesetzt und das Reaktionsgemisch wird bei -30°C 30 Minuten lang gerührt. Danach wird eine Lösung des entsprechenden Dialkylchlorphosphin (14,8 mmol in 10 ml THF) langsam mittels Tropftrichter zugegeben (bei -30°C), auf Raumtemperatur erwärmt, weiter erwärmt auf ca. 50°C und für 60 Minuten gerührt. Nach Kühlung in einem Eisbad wird eine entgaste wässrige Ammoniumchloridlösung zugegeben, kurzzeitig gerührt und die organische Phase wird abgetrennt. Die wässrige Schicht wird mit Toluol (2 x 20 ml) extrahiert und die kombinierten organischen Schichten werden bei 45°C unter Vakuum eingeengt. Das Produkt wird aus Diethylether umkristallisiert und ergibt ein farbloses festes Produkt.

### Herstellung von 2-(Dicyclohexylphosphino)-1-(2-methoxyphenyl)-1H-imidazol (L1)

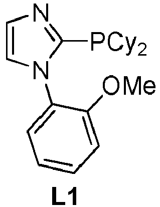

Entsprechend der obigen allgemeinen Vorschrift wurde L1 aus 1-(2-Methoxyphenyl)-1 H-imidazol (2,35 g, 13,5 mmol) and Dicyclohexylchlorphosphin (3,43 g, 1,8 mmol) synthetisiert. Es wurde ein farbloser Feststoff erhalten (3,40 g, 68% Ausbeute).

Mit dem Liganden L1 wurde eine Ruthenium-katalysierte Hydroaminomethylierung von 1-Octen mit Ammoniak nach folgendem Reaktionsschema durchgeführt: 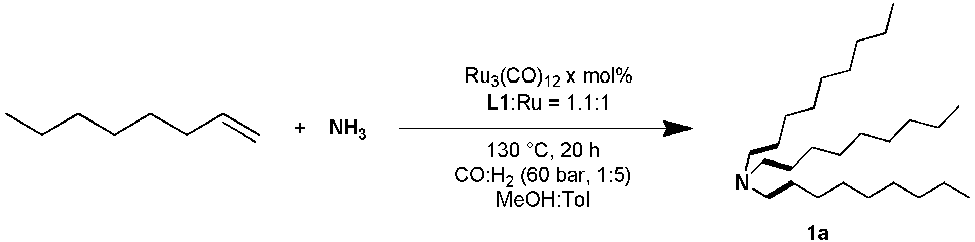 Als Katalysator diente Ru₃(CO)₁₂ kombiniert mit dem Liganden L1.
Versuchsbedingungen, Ausbeuten und Selektivitäten sind der Tabelle 1 zu entnehmen.

(*n*/*iso*) = das Verhältnis von linearem (n) zum verzweigten (iso) Produkt, iso ist die Summe aller verzweigten Alkylketten

**Tabelle 1: Variation der Ammoniakquelle**

| | | Ausbeute [%] | | | |
|---|---|---|---|---|---|
| Bsp. | Bedingungen | Octene | Octan | prim. & sek. Amine | tert. Amine (*n*/*iso*) |
| 1 | NH₃(7 N in MeOH, 10 mL, 70 mmol) | 15 | 9 | 4 | 61 (90:10) |
| 2 | NH₃(7 N in MeOH, 2 mL, 14 mmol) | 9 | 9 | 3 | 65 (88:12) |
| 3 | NH₃(0,5 N in Dioxan, 20 mL, 10 mmol) | 7 | 10 | 3 | 66 (89:11) |
| 4 | Harnstoff (1,32 g, 20 mmol) | 6 | 7 | 2 | 51 (80:20) |
| 5 | NH₃ (28-30 w% in H₂O, 1 mL, ≈ 16 mmol) | 10 | 8 | 1 | 63 (87:13) |
| 6 | NH₃(28-30 w% in H₂O, 1 mL, ≈ 16 mmol) | 8 | 9 | 2 | 71 (87:13) |
| 7 | NH₃ (0,5g, ≈ 29 mmol) | 9 | 9 | 2 | 72 (88:12) |

*Beispiel 1 (Tabelle 1):* Ein 100-mL Autoklav wird befüllt mit Ru₃(CO)₁₂ (6,4 mg; 0,1 mol%), dem Liganden L1 (12,2 mg; 0,33 mol%), Toluol (10 mL), 1-Octen (3,1 mL; 2,2 g; 20 mmol) und NH₃ (7 N in MeOH, 10 mL, 70 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Das tertiäre Amin wird mit einer hohen Ausbeute (61 %) und hoher linearer Selektivität *(n:iso =* 90:10) gebildet. Primäre und sekundäre Amine werden nur zu insgesamt 4 % gebildet.

*Beispiel 2 (Tabelle 1):* Ein 100-mL Autoklav wird befüllt mit Ru₃(CO)₁₂ (6,4 mg; 0,1 mol%), dem Liganden L1 (12,2 mg; 0,33 mol%), Methanol (10 mL), Toluol (10 mL), 1-Octen (3,1 mL; 2,2 g; 20 mmol) und NH₃ (7 N in MeOH, 2 mL, 14 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Das tertiäre Amin wird mit einer hohen Ausbeute (65 %) und hoher linearer Selektivität (n:iso = 88:12) gebildet. Primäre und sekundäre Amine werden nur zu insgesamt 3 % gebildet.

*Beispiel 3 (Tabelle 1)*: Ein 100-mL Autoklav wird befüllt mit Ru₃(CO)₁₂ (6,4 mg; 0,1 mol%), dem Liganden L1 (12,2 mg; 0,33 mol%), 1-Octen (3,1 mL; 2,2 g; 20 mmol) und NH₃ (0,5 N in Dioxan, 20 mL, 10 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Das tertiäre Amin wird mit einer hohen Ausbeute (66 %) und hoher linearer Selektivität (n:iso = 89:11) gebildet. Primäre und sekundäre Amine werden nur zu insgesamt 3 % gebildet.

Mit den Beispielen 1 bis 3 kann gezeigt werden, dass das Verfahren sehr effizient und selektiv in der Hydroaminomethylierung ist.

*Beispiel 4 (Tabelle 1)*: Ein 100-mL Autoklav wird befüllt mit Ru₃(CO)₁₂ (6,4 mg; 0,1 mol%), dem Liganden L1 (12,2 mg; 0,33 mol%), Methanol (10 mL), Toluol (10 mL), 1-Octen (3,1 mL; 2,2 g; 20 mmol) und Harnstoff (1,32 g, 20 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Das tertiäre Amin wird mit einer im Vergleich zu den übrigen Beispielen der Tabelle 1 geringeren Ausbeute (51 %) und niedrigerer linearer Selektivität (n:iso = 80:20) gebildet. Primäre und sekundäre Amine werden nur zu insgesamt 2 % gebildet.

*Beispiel 5 (Tabelle 1)*: Ein 100-mL Autoklav wird befüllt mit Ru₃(CO)₁₂ (6,4 mg; 0,1 mol%), dem Liganden L1 (12,2 mg; 0,33 mol%), Methanol (10 mL), Toluol (10 mL), 1-Octen (3,1 mL; 2,2 g; 20 mmol) und wässrigem Ammoniak (NH₃ 28-30 Massen% in H₂O, 1 mL, ≈ 16 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Das tertiäre Amin wird mit einer hohen Ausbeute (63 %) und hoher linearer Selektivität *(n:iso =* 87:13) gebildet. Primäre und sekundäre Amine werden nur zu insgesamt 1 % gebildet.

*Beispiel 6 (Tabelle 1)*: Ein 100-mL Autoklav wird befüllt mit Ru₃(CO)₁₂ (12,8 mg; 0,2 mol%), dem Liganden L1 (24,8 mg; 0,66 mol%), Methanol (10 mL), Toluol (10 mL), 1-Octen (1,6 mL; 1,1 g; 10 mmol) und wässrigem Ammoniak (NH₃ 28-30 Massen% in H₂O, 1 mL, ≈ 16 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*iso*-Octan) wird die Reaktionsmischung gaschromatographisch analysiert.

Das tertiäre Amin wird mit einer hohen Ausbeute (71 %) und hoher linearer Selektivität (n:iso = 87:13) gebildet. Primäre und sekundäre Amine werden nur zu insgesamt 2 % gebildet.

Bei Einsatz von wässrigem Ammoniak wird eine hohe Ausbeute an tertiären Aminen, jedoch eine geringfügig niedrigere lineare Selektivität im Vergleich zu den Beispielen 1 bis 3 beobachtet (Beispiele 5 und 6). Eine Steigerung der Ausbeute ist durch Erhöhung der Katalysator-Menge möglich (vgl. Beispiel 6).

*Beispiel 7 (Tabelle 1)*: Ein 100-mL Autoklav wird befüllt mit Ru₃(CO)₁₂ (12,8 mg; 0,2 mol%), dem Liganden L1 (24,8 mg; 0,66 mol%), Methanol (10 mL), Toluol (10 mL), 1-Octen (1,6 mL; 1,1 g; 10 mmol) und NH₃ (0.5 g, ≈ 29 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*iso*-Octan) wird die Reaktionsmischung gaschromatographisch analysiert.

Das tertiäre Amin wird mit einer hohen Ausbeute (71 %) und hoher linearer Selektivität *(n:iso =* 87:13) gebildet. Primäre und sekundäre Amine werden nur zu insgesamt 2 % gebildet. Dieses Ergebnis ist vergleichbar mit dem Ergebnis, welches unter Einsatz von wässrigem Ammoniak erzielt wurde.

Aus Kostengründen und aufgrund der verbesserten Handhabung wurden die nachfolgenden Experimente deshalb mit wässrigem Ammoniak durchgeführt.

Nachfolgend wurde der Einfluss des Lösungsmittels untersucht.

Für sämtliche *Beispiele 8 bis 17* gelten folgende Angaben:
Ein 100-mL Autoklav wird befüllt mit Ru₃(CO)₁₂ (12,8 mg; 0,2 mol%), dem Liganden L1 (24,8 mg; 0,66 mol%), 20 mL Lösungsmittel wie in der Tabelle 2 angegeben, 1-Octen (1,6 mL; 1,1 g; 10 mmol) und wässrigem Ammoniak NH₃ (28-30 w% in H₂O, 1 mL, ≈ 16 mmol). Synthesegas (10 bar CO / 50 bar H₂) wird aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (*iso*-Octan) wird die Reaktionsmischung gaschromatographisch analysiert.

Die eingesetzten Lösungsmittel und erzielten Ausbeuten und Selektivitäten finden sich in der Tabelle 2.

**Tabelle 2: Variation des Lösungsmittels**

| | | Ausbeute [%] | | | |
|---|---|---|---|---|---|
| Bsp. | Lösungsmittel | Octene | Octan | prim. & sek. Amine | tert. Amine (*n*/*iso*) |
| 8 | Toluol:Methanol | 8 | 9 | 2 | 71 (87:13) |
| 9 | Toluol | 5 | 9 | 0 | 62 (90:10) |
| 10 | Methanol | 7 | 8 | 0 | 62 (82:18) |
| 11 | *iso*-Propanol | 9 | 9 | 5 | 60 (90:10) |
| 12 | Ethanol | 14 | 8 | 1 | 56 (85:15) |
| 13 | Acetonitril | 20 | 6 | 0 | 54 (89:11) |
| 14 | Tetrahydrofuran (THF) | 4 | 7 | 8 | 74 (89:11) |
| 15 | THF:Toluol | 3 | 7 | 4 | 80 (90:10) |
| 16 | THF:Methanol | 13 | 7 | 0 | 77 (88:12) |
| 17 | THF:*iso*-Propanol | 7 | 9 | 8 | 72 (90:10) |

Verglichen mit der 1:1 Mischung von Toluol und Methanol (Tabelle 2, Beispiel 8), ergaben Toluol und Methanol allein geringere Ausbeuten am sekundären Amin, wobei bei Toluol als Lösungsmittel die lineare Selektivität mit 90:10 höher war als bei Methanol 82:18. Andere alkoholische Lösungsmittel wie *i*so-Propanol und Ethanol ergaben geringere Ausbeuten, aber bessere Regioselektivitäten als bei Einsatz von Methanol als Lösungsmittel (Tabelle 2, Beispiele 11 und 12). Bei Einsatz von Acetonitril wurde die Bildung großer Mengen von isomerisierten Octenen beobachtet (Tabelle 2, Beispiel 13). Höhere Ausbeuten und eine gute Regioselektivität konnte mit THF erreicht werden (Tabelle 2, Beispiel 14). Beste Ergebnisse bei der Ausbeute und der Regioselektivität wurden mit einem 1:1 Gemisch von THF und Toluol erhalten (Tabelle 2, Beispiele 15 - 17).

Des Weiteren wurde der Einfluss von Druck und Temperatur untersucht.

Für sämtliche *Beispiele 18-25* gelten folgende Angaben:
Ein 100-mL Autoklav wird befüllt mit Ru₃(CO)₁₂ (12,8 mg; 0,2 mol%), dem Liganden L1 (24,8 mg; 0,66 mol%), THF (10 mL), Toluol (10 mL), 1-Octen (1,6 mL; 1,1 g; 10 mmol) und wässrigem Ammoniak NH₃ (28-30 w% in H₂O, 1 mL, ≈ 16 mmol). Synthesegas (10 bar CO / 50 bar H₂) wird aufgepresst. Die Reaktionsmischung wird auf 130 °C bzw. 120°C erwärmt und 20 Stunden bei dieser Temperatur gerührt. Danach wird der Autoklav abgekühlt und Gas abgelassen. Nach der Zugabe des internen Standards (*i*so-Octan) wird die Reaktionsmischung gaschromatographisch analysiert.

Die einzelnen Drücke und Temperaturen, sowie die erzielten Ausbeuten und Selektivitäten finden sich in der Tabelle 3.

**Tabelle 3: Variation von Druck und Temperatur**

| | | Ausbeute [%] | | | |
|---|---|---|---|---|---|
| Bsp. | CO:H₂ [bar] / Temperatur | Octene | Octan | prim. & sek. Amine | tert. Amine (*n*/*iso*) |
| 18 | 10:50 / 130 °C | 3 | 3 | 4 | 80 (90:10) |
| 19 | 7:35 / 130 °C | 3 | 3 | 10 | 76 (91:9) |
| 20 | 5:25 / 130 °C | 8 | 8 | 22 | 58 (93:7) |
| 21 | 20:40 / 130 °C | 10 | 10 | 3 | 77 (89:11) |
| 22 | 10:40 / 130 °C | 4 | 4 | 5 | 79 (90:10) |
| 23 | 10:30 / 130 °C | 7 | 7 | 4 | 72 (92:8) |
| 24 | 10:50 / 120 °C | 4 | 4 | 6 | 79 (90:10) |
| 25 | 10:40 / 120 °C | 5 | 5 | 4 | 79 (90:10) |

Bei niedrigeren Drücken wurden verstärkt primäre und sekundäre Amine gebildet (Tabelle 3, Beispiele 18 - 20). Höhere Konzentrationen an CO führten zu mehr internen Alkenen als Nebenprodukt, vermutlich da die Reduktionsfähigkeit von Ruthenium reduziert wird (Tabelle 3, Beispiele 21 und 22). Die Reduzierung des Partialdruckes von Wasserstoff führte zu geringeren Aminausbeuten (Tabelle 3, Beispiel 23). Die Temperatur konnte auf 120 °C gesenkt werden, ohne die Reaktion maßgeblich zu beeinflussen (Tabelle 3, Beispiele 24 und 25).

Die Eignung des Verfahrens für andere Verbindungen, welche mindestens eine Doppelbindung enthalten, wurde an folgendem System untersucht:

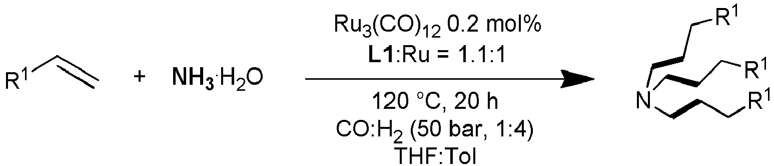

Für sämtliche *Beispiele 26 bis 38* gelten folgende Angaben:
Ein 100-mL Autoklav wird befüllt mit Ru₃(CO)₁₂ (12,8 mg; 0,2 mol%), dem Liganden L1 (24,8 mg; 0,66 mol%), THF (10 mL), Toluol (10 mL), 10 mmol der jeweiligen Verbindung, welche mindestens eine Doppelbindung enthält, wie angegeben im nachfolgenden Schema, und wässrigem Ammoniak NH₃ (28-30 w% in H₂O, 1 mL, ≈ 16 mmol). Synthesegas (10 bar CO / 40 bar H₂) wird aufgepresst. Die Reaktionsmischung wird auf 120 °C erwärmt und 20 Stunden bei dieser Temperatur gerührt. Danach wird der Autoklav abgekühlt und Gas abgelassen. Nach der Zugabe des internen Standards (*iso*-Octan) wird die Reaktionsmischung gaschromatographisch analysiert.

Die Ergebnisse aus der Substratvariation sind nachfolgend dargestellt.

Im Vergleich zu der Umsetzung von 1-Octen läuft die Umsetzung der kürzerkettigeren Alkene 1-Penten und 1-Hexen zu den Produkten 28 und 29 in vergleichbaren Ausbeuten, aber mit leicht verbesserter Regioselektivität ab (*n:iso* = 92:8). In Übereinstimmung hiermit sinkt die Regioselektivität bei Einsatz von 1-Decen zu 87:13 ab (30). Die Produkte der Reaktionen von Allylcyclohexen (31) und Allylbenzen (32) wurden ebenfalls in guten Ausbeuten erhalten, aufgrund der Isomerisierung der Doppelbindung wurde bei Umsetzung des Allylbenzens (32) jedoch eine geringere Regioselektivität beobachtet. Eine gute Ausbeute und gute Regioselektivität wurde bei der Reaktion von 3,3-Dimethylbut-1-en erreicht (33). Die Umsetzung von cyclischen Alkenen wie Cyclohexen musste bei einer höheren Temperatur durchgeführt werden (160°C) um eine Ausbeute von 47% zu erreichen (34). Auch für die Umsetzung von α-Methylstyren war eine erhöhte Temperatur notwendig (35). Gute Resultate konnten auch für funktionalisierte Alkene erreicht werden. Beispielsweise konnte das Hydroxyl-substituierte Produkt 36 mit einer Ausbeute von 76 % und einer hohen Regioselektivität von 99:1 hergestellt werden (36). Ester- und Nitrilgruppen wurden unter den gewählten Reaktionsbedingungen ebenfalls toleriert, die Umsetzungen lieferten die entsprechenden tertiären Amine mit einer Regioselektivität von 90:10 bzw. 83:17.

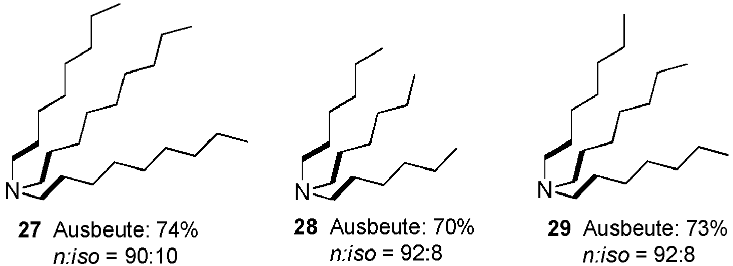

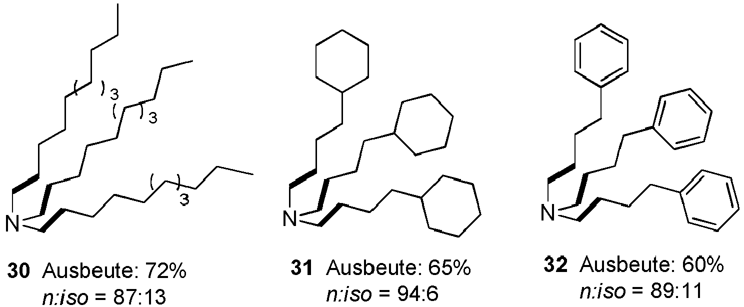

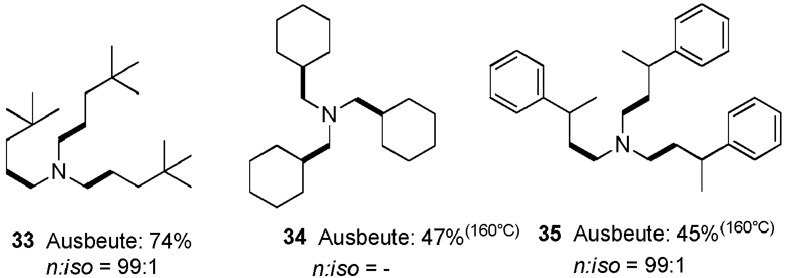

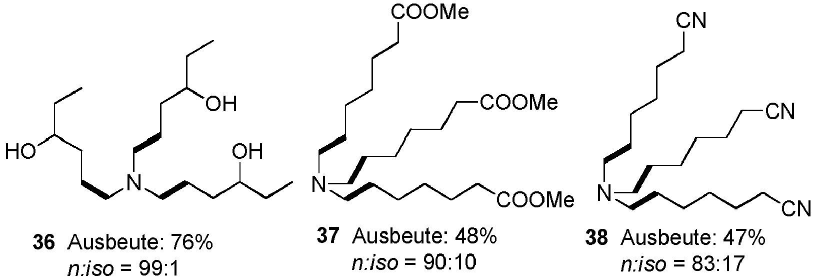

Somit konnte gezeigt werden, dass das beanspruchte Verfahren auf eine Vielzahl von Substrate angewendet und verschiedenste funktionelle Gruppen toleriert werden. Wie die Versuchsergebnisse zeigen, wird die gestellte Aufgabe durch das erfindungsgemäße Verfahren gelöst.

Es ist somit erstmalig gelungen, tertiäre Amine durch ein Hydroaminomethylierungsverfahren in Gegenwart von Ruthenium-Katalysatoren in guten bis sehr guten Ausbeuten herzustellen. Dieses Verfahren sollte direkt von Ammoniak oder einer Ammoniakquelle ausgehen.

## Patentansprüche

1. Verfahren zur Hydroaminomethylierung von Verbindungen, welche mindestens eine Doppelbindung enthalten, **dadurch gekennzeichnet, dass** wenigstens eine Verbindung, welche mindestens eine Doppelbindung enthält, mit Ammoniak oder einer Ammoniakquelle unter Zuführung von H₂ und CO in Gegenwart eines katalytischen Systems aus einem Ruthenium-Komplex und mindestens einem Liganden umgesetzt wird, wobei der Ligand einen organischen Phosphor-Liganden einer der allgemeinen Formeln (1) bis (8) darstellt: 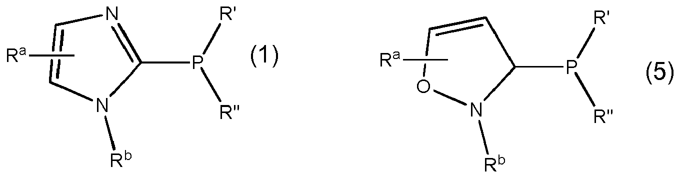 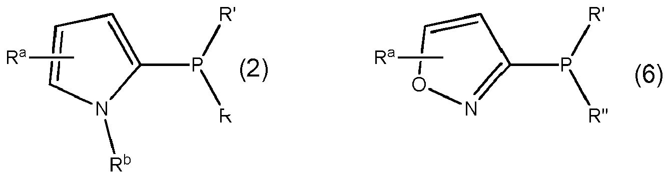 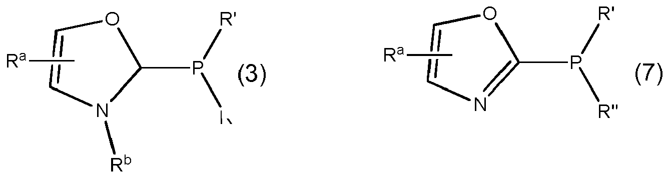 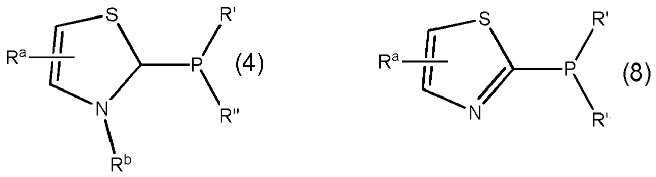 worin
R' und R" gleich oder verschieden sind und für einen Rest stehen, der ausgewählt ist aus der Gruppe bestehend aus:
(C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
wobei die genannten Alkyl-, Heteroalkyl-, Cycloalkyl-, Heterocycloalkyl-, Aryl- und Heteroarylgruppen optional ein oder mehrfach substituiert sind und
R^{a} und R^{b} ausgewählt sind aus: Wasserstoff, Hydroxy, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
wobei die genannten Alkyl-, Heteroalkyl-, Cycloalkyl-, Heterocycloalkyl-, Aryl- und Heteroarylgruppen optional ein oder mehrfach substituiert sind und
und wobei R^{a} auch zur Bildung eines größeren kondensierten Ringes befähigt ist.

2. Verfahren gemäß Anspruch 1, **dadurch gekennzeichnet, dass** der Ligand die allgemeine Formel (1) oder (2) aufweist.

3. Verfahren gemäß Anspruch 1 oder 2, **dadurch gekennzeichnet, dass** R' und/oder R" unabhängig voneinander ausgewählt ist aus Cyclohexyl, iso-Propyl und Phenyl.

4. Verfahren gemäß einem der Ansprüche 1 oder 2, **dadurch gekennzeichnet, dass** der Ligand ausgewählt ist aus der Gruppe bestehend aus:
2-(Dicyclohexylphosphino)-1-(2-methoxyphenyl)-1H-imidazol (L1),
2-(Dicyclohexylphosphino)-1-methyl-1 H-imidazol (L2),
2-(Dicyclohexylphosphino)-1-phenyl-1 H-imidazol (L3),
2-(Di*iso*propylphosphino)-1-(2-methoxyphenyl)-1H-imidazol (L4),
2-(Diphenylphosphino)-1-(2-methoxyphenyl)-1 H-imidazol (L5),
2-(Dicyclohexylphosphino)-1-mesityl-1 H-imidazol (L6),
2-(Dicyclohexylphosphino)-1-phenyl-1 H-pyrrol (L7),
2-(Dicyclohexylphosphino)-1-(2-methoxyphenyl)-1H-pyrrol (L8),
2-(Dicyclohexylphosphino)-1-methyl-1 H-benzo[d]imidazol (L9).

5. Verfahren gemäß einem der Ansprüche 1 bis 4, **dadurch gekennzeichnet, dass** der Ruthenium-Katalysator in situ ausgehend von einem Vorkomplex gebildet wird, wobei als Rutheniumquelle Ruthenium-enthaltende Salze und Komplexe als Vorstufe verwendet werden, die Rutheniumcarbonylhydrid-Komplexe bilden.

6. Verfahren gemäß einem der Ansprüche 1 bis 5, **dadurch gekennzeichnet, dass** die Verbindung, welche mindestens eine Doppelbindung enthält, ausgewählt ist aus α-Alkenen, internen lineare Alkenen, interne verzweigten Alkenen, Cycloalkenen, aromatischen Olefinen, Polyenen, ungesättigten Alkoholen, ungesättigten Ethern, ungesättigten Aminen, ungesättigten Estern, ungesättigten Carbonsäuren, ungesättigten Amiden, ungesättigten Urethanen, ungesättigten Halogeniden, ungesättigten Aldehyden, ungesättigten Ketonen und ungesättigten Epoxiden sowie Mischungen aus diesen.

7. Verfahren gemäß einem der Ansprüche 1 bis 6, **dadurch gekennzeichnet, dass** in dem Verfahren weniger als 10 Massen-%, vorzugsweise weniger als 2 Massen-% und insbesondere kein Lösungsmittel eingesetzt wird, wobei die Massen-% auf das ganze Reaktionsgemisch bezogen sind.

8. Gemische erhältlich durch ein Verfahren nach einem der Ansprüche 1 bis 7.

9. Verwendung eines organischen Phosphor-Liganden einer der allgemeinen Formeln (1) bis (8) 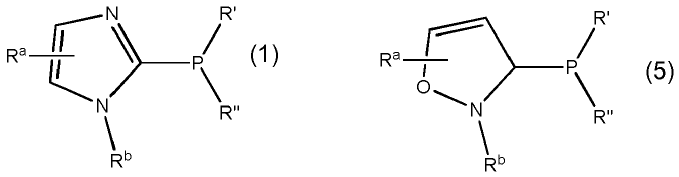 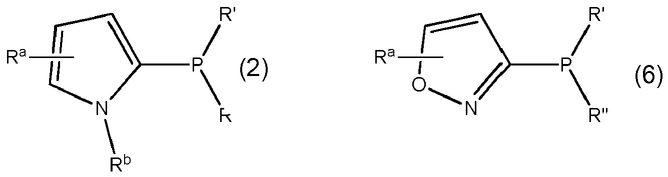 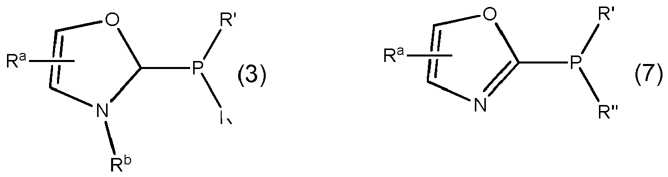 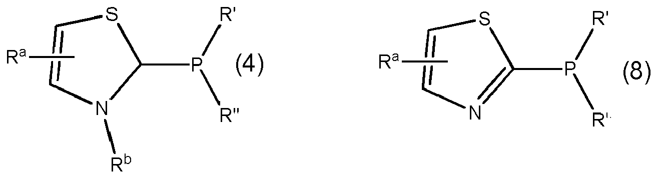 worin
R' und R" gleich oder verschieden sind und für einen Rest stehen, der ausgewählt ist aus der Gruppe bestehend aus:
(C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
wobei die genannten Alkyl-, Heteroalkyl-, Cycloalkyl-, Heterocycloalkyl-, Aryl- und Heteroarylgruppen optional ein oder mehrfach substituiert sind und
R^{a} und R^{b} ausgewählt sind aus: Wasserstoff, Hydroxy, (C₁-C₁₂)-Alkyl, (C₁-C₁₂)-Heteroalkyl, (C₄-C₁₄)-Aryl, (C₄-C₁₄)-Aryl-(C₁-C₁₂)-Alkyl, (C₄-C₁₄)-Aryl-O-(C₁-C₁₂)-Alkyl, (C₃-C₁₄)-Heteroaryl, (C₃-C₁₄)-Heteroaryl-(C₁-C₁₂)-Alkyl, (C₃-C₁₂)-Cycloalkyl, (C₃-C₁₂)-Cycloalkyl-(C₁-C₁₂)-Alkyl, (C₃-C₁₂)-Heterocycloalkyl, (C₃-C₁₂)-Heterocycloalkyl-(C₁-C₁₂)-Alkyl, O-(C₁-C₁₂)-Alkyl, O-(C₁-C₁₂)-Heteroalkyl, O-(C₄-C₁₄)-Aryl, O-(C₄-C₁₄)-Aryl-(C₁-C₁₄)-Alkyl, O-(C₃-C₁₄)-Heteroaryl, O-(C₃-C₁₄)-Heteroaryl-(C₁-C₁₄)-Alkyl, O-(C₃-C₁₂)-Cycloalkyl, O-(C₃-C₁₂)-Cycloalkyl-(C₁-C₁₂)-Alkyl, O-(C₃-C₁₂)-Heterocycloalkyl, O-(C₃-C₁₂)-Heterocycloalkyl-(C₁-C₁₂)-Alkyl,
wobei die genannten Alkyl-, Heteroalkyl-, Cycloalkyl-, Heterocycloalkyl-, Aryl- und Heteroarylgruppen optional ein oder mehrfach substituiert sind und
und wobei R^{a} auch zur Bildung eines größeren kondensierten Ringes befähigt ist,
bei der Umsetzung von Ammoniak oder einer Ammoniakquelle mit Verbindungen, welche mindestens eine Doppelbindung enthalten.

10. Verwendung eines organischen Phosphorliganden aus der Gruppe, die gebildet wird durch
2-(Dicyclohexylphosphino)-1-(2-methoxyphenyl)-1H-imidazol,
2-(Dicyclohexylphosphino)-1-methyl-1 H-imidazol,
2-(Dicyclohexylphosphino)-1-phenyl-1 H-imidazol,
2-(Di*iso*propylphosphino)-1-(2-methoxyphenyl)-1H-imidazol,
2-(Diphenylphosphino)-1-(2-methoxyphenyl)-1H-imidazol,
2-(Dicyclohexylphosphino)-1-mesityl-1H-imidazol,
2-(Dicyclohexylphosphino)-1-phenyl-1H-pyrrol,
2-(Dicyclohexylphosphino)-1-(2-methoxyphenyl)-1H-pyrrol und
2-(Dicyclohexylphosphino)-1-methyl-1 H-benzo[d]imidazol
bei der Umsetzung von Ammoniak oder einer Ammoniakquelle mit Verbindungen, welche mindestens eine Doppelbindung enthalten.
